# EUROPEAN PATENT SPECIFICATION

(11) **EP 1 401 402 B1**
(45) Date of publication and mention of the grant of the patent: **07.09.2016**
(21) Application number: 02737142.6
(22) Date of filing: 28.05.2002
(51) Int. Cl.: A61K 31/343, A61K 31/4375, A61K 31/545, A61K 31/65, A61K 31/7048, A61K 9/00, A61K 9/16, A61K 9/20

(54) **ENHANCEMENT OF ORAL BIOAVAILABILITY OF NON-EMULSIFIED FORMULATION OF CEFDITOREN PIVOXIL WITH LECITHIN**
ERHÖHUNG DER ORALEN BIOVERFÜGBARKEIT EINER NICHT-EMULGIERTEN FORMULIERUNG CEFDITOREN PIVOXIL MIT LECITHIN
AMELIORATION DE LA BIODISPONIBILITE ORALE DE PREPARATION NON EMULSIONNEE DE CEFDITORENE PIVOXIL AVEC DE LA LECITHINE

(30) Priority: 29.05.2001 US 294141 P; 29.05.2001 US 867353
(43) Date of publication of application: 31.03.2004
(73) Proprietor: Takeda Pharmaceuticals U.S.A., Inc., Deerfield, IL 60015 (US)
(72) Inventor: TANEJA, Rajneesh, Libertyville, IL 60048 (US); BRINKER, Dale, Antioch, IL 60002 (US); BRISKIN, Jacqueline, Buffalo Grove, IL 60089 (US); VISHWASRAO, Dilip, Waukegan, IL 60085 (US); APONTE, Roberto, Grayslake, IL 60030 (US); GUPTA, Pramod, Gurnee, IL 60031 (US)
(74) Representative: Modiano, Micaela Nadia
(86) International application number: PCT/US2002/016428
(87) International publication number: WO 2002/096354

(56) References cited:
- EP-A1- 1 190 720
- WO-A-90/15609
- WO-A1-99/26632
- DATABASE WPI Derwent Publications Ltd., London, GB; AN 1987-061184, XP002094704 'Pharmaceutical antifungal composition' & JP 62 016 431 A 24 January 1987

## Description

### Field of the instant disclosure.

The instant disclosure is directed to a method for enhancing the oral bioavailability of a prodrug ester by formulating the ester as a non-emulsified formulation with lecithin; as well as a pharmaceutical composition of at least one antibiotic and lecithin in a non-emulsified formulation; a method of treating infections with the non-emulsified formulation, and a method for preparing tablets by direct compression of blends of drugs with lecithin. Non-emulsified formulations include solids, tablets, capsules, lozenges, suspensions, elixirs and solutions, and exclude emulsions, liposomes, lipid matrix systems and micro-emulsions. A suitable prodrug ester is a cephalosporin β-lactam antibiotic such as cefditoren pivoxil, and a suitable non-emulsified formulation is a solid formulation.

### Background of the Invention

The oral bioavailability of a number of drugs can be enhanced by the synthesis of their pro-drug esters. Such chemical modification alters the lipophilicity of these compounds which makes them more suitable candidates for passive diffusion across the gastrointestinal tract mucosa, thus improving their oral absorption. Once absorbed, these pro-drugs undergo hydrolysis to generate the parent compound which is therapeutically active. A number of classes of compounds including cephalosporins have benefitted from this approach.

Most cephalosporins are characterized by a dipeptide-like structure containing a free carboxyl group that is ionized at the physiological intestinal pH. Many cephalosporins may also contain a relatively basic amino group. The resulting polarity makes these cephalosporins a poor candidate for oral administration. One approach to enhancing oral bioavailability of cephalosporins is the esterification of the carboxylic acid group in the 4- position. The derivatives so formed have reduced polarity and can be absorbed by passive diffusion.

Cefditoren is a cephalosporin β-lactam antibiotic that exhibits potent antibacterial activities against both Gram positive and Gram negative bacteria. However, its oral absorption is limited. Cefditoren synthesis is disclosed in U.S. Patent Nos. 4,839,350 and 4,918,068; and an injectable cefditoren preparation is disclosed in U.S. Patent No. 5,595,986. Cefditoren pivoxil, synthesized by forming a pivaloyloxymethyl (pivoxil) ester with cefditoren at the carboxylic acid moiety, exhibits better oral absorption and is quickly hydrolyzed to cefditoren by enzymatic esterases upon absorption. However, the oral bioavailability of this compound is low, as the pro-drug ester may undergo premature hydrolysis even before it can be absorbed, which results in a parent compound with low oral bioavailability. Cefditoren pivoxil is degraded in the intestinal tract into cefditoren and pivaloyloxymethyl alcohol. Additionally, cefuroxime axetil, cefetamet pivoxil and cefpodoxime proxetil undergo hydrolysis in the human intestinal juices. Addition of a water-soluble casein salt to cefditoren pivoxil has been disclosed in U.S. Patent No. 5,958,915 as a method for enhancing solubility of the drug.

Esterase enzymes are hydrolases that split ester bonds and are involved in the metabolism of a number of compounds used as drugs in humans. Three esterases have been found to be responsible for the hydrolysis of exogenous compounds, namely- carboxylesterases, cholinesterases and aryl-esterases. Previously conducted studies with cefditoren pivoxil in rats have demonstrated that carboxylesterases are primarily involved in the metabolism of the ester side chain and that cholinesterases may be partially involved.

A number of approaches have been tried for prevention of the premature de-esterification of pro-drug esters. For example, esterase inhibitor adjuncts such as *p-*nitrophenyl phosphate and bis- *p*-nitrophenylphosphate for carboxylesterases; neostigmine for cholinesterases or p-hydroxymercuribenzoate for arylesterases have been utilized. Another approach utilizes fruit extract esters for reducing the esterase-mediated degradation of the pro-drug esters. In yet another approach, cefpodoxime proxetil has been formulated as a sub-micro-emulsion to protect the pro-drug from cholinesterase enzyme. However, improved methods for enhancing oral bioavailability of cephalosporins would still represent a significant advancement in the art.

We have now discovered that the use of lecithin with cefditoren pivoxil in solid formulation greatly enhances oral bioavailability of the drug.

Historically, the term lecithin, originated from the Greek word 'lekithos', was used for the phosphorus containing lipids from egg yolk. Later, this term was only used for one defined phospholipid - phosphatidylcholine. This term is still commonly used in the scientific literature, where lecithin stands for 1,2-diacyl-sn-glycero-3-phosphatidylcholine. The commercially available lecithin used in pharmaceuticals and food products is a term used for the complex mixture of neutral lipids (predominantly triglycerides, a small amount of free fatty acids and sterols), polar lipids (phospho- and glycolipids) and carbohydrates. The principal phospholipids are PC (phosphatidyl-choline), PE (phosphatidyl-ethanolamine), and PI (phosphatidyl-inositol). Some of the sources for lecithin include egg, soybeans, rapeseed, and safflower.

Lecithins are widely used in the pharmaceutical industry as dispersing, emulsifying and stabilizing agents. They are used in formulations meant for intravenous, intramuscular, topical, oral and rectal administration. U.S. Patent No. 5,319,116 discloses a method for preparation of lecithin fractions for pharmacological use. Lecithins have been widely investigated for their role in enhancing the bioavailability of drugs. For example, U.S. Patent No. 5,098,606 discloses the use of lecithin as an emulsifying agent for enhancing the bioavailability of cephalosporins; U.S. Patent No. 5,651,991 discloses fatty emulsions of particles useful for drug delivery wherein the surface layer of the emulsion consists of lecithin; U.S. Patent No. 6,113,921 discloses pharmaceutical compositions for topical or transdermal applications including a phospholipid emulsifier such as lecithin; U.S. Patent No. 6,127,349 discloses a method of enhancing bioavailability of drugs including antibiotics by conjugation with phospholipids and Fagerholm *et al.* have disclosed a "lipid matrix drug delivery system" consisting of phosphotidyl choline and medium chain monoacyl glycerol in J. Pharm. Pharmacol., Vol. 50 (5) pp. 467-473.

Moreover, Crauste-Manciet *et al.* have disclosed a method for protecting cefpodoxime proaxetil from degradation in the presence of carboxylesterase *enzyme* as a formulation of a micro-emulsion using soy lecithin as an emulsifying agent in the International Journal of Pharmaceutics, Vol. 165, (1998) pp. 97-106. In the method, cefpodoxime proaxetil was dissolved in a co-solvent and this mixture was subsequently dissolved in soybean oil or a medium chain triglyceride oil. Lecithin, when used, was dissolved in oil phases and polysorbate 20 (nonionic emulsifier) when used, was dissolved in the aqueous phase. Both phases were heated to 60°C, mixed and emulsified by the phase inversion method using a high shear mixer.

Additionally, Burns *et al.* disclose a synthesis of mixed micelles using short chain lecithin/triglyceride combination in The Journal of Biological Chemistry, Vol. 256, (1981), pp. 2716-2722. The synthesis requires co-solubilization of both lipids in benzene and or/chloroform, solvent removal under N₂, and evacuation at low pressure for at least two hours, followed by addition of aqueous solution, and incubation for four hours at room temperature. Lipase hydrolysis rates of the triglyceride in these particles by phospholipase enzyme were 0.3-0.5 times those of triglyceride emulsions alone.

JPS6216431 (A) is directed to a pharmaceutical composition, containing an antibiotic substance and a phospholipid and capable of improving the absorbability of the antibiotic substance.

WO9926632 (A1) discloses that when phospholipids are combined with antibiotics (e.g. ampicillin, ceftazidime, or piperacillin) the effect of the antibiotic is enhanced by the presence of the phospholipid.

However, a simplified drug delivery system which enhances the oral bioavailability of cefditoren pivoxil without using complex formulation techniques, such as emulsions, micro-emulsions or matrices, would be desirable.

### Brief Summary of the Invention

Lecithin has been utilized in emulsion, micro-emulsion and liposomal drug formulations. However, we have now surprisingly found that when lecithin is combined with cefditoren pivoxil in a nonemulsified oral formulation, such as a solid formulation, without addition of emulsifiers, degradation of the cefditoren pivoxil is greatly impeded. In particular, the effectiveness of cefditoren pivoxil can be greatly enhanced in a solid lecithin-containing formulation.

More in particular, the invention encompasses various embodiments as defined in claims 1, 2, 3, 4, 6 and 9 as attached.

The invention encompasses a method of enhancing the oral bioavailability of a cefditoren pivoxil or a pharmaceutically acceptable salt thereof comprising the step of formulating cefditoren pivoxil or a pharmaceutically acceptable salt thereof in a nonemulsified formulation with lecithin.

The invention also encompasses a method of enhancing the oral bioavailability of cefditoren pivoxil or a pharmaceutically acceptable salt thereof comprising the step of formulating cefditoren pivoxil or a pharmaceutically acceptable salt thereof in a solid formulation with lecithin.

The invention is also directed to a pharmaceutical composition comprising: cefditoren pivoxil or a pharmaceutically acceptable salt thereof and lecithin, wherein said composition is not emulsified.

The invention is also directed to a pharmaceutical composition comprising: cefditoren pivoxil or a pharmaceutically acceptable salt thereof and lecithin, wherein said composition is a solid.

A presently preferred pharmaceutical composition contains cefditoren pivoxil or a pharmaceutically acceptable salt thereof; and lecithin, wherein said composition is a solid.

The invention is also directed to cefditoren pivoxil or a pharmaceutically acceptable salt thereof for use in a method of treating infections comprising the step of administering to a patient in need of such treatment a therapeutically effective amount of a solid formulation of cefditoren pivoxil or a pharmaceutically acceptable salt thereof and lecithin.

The invention is also directed to a method for preparing tablets by direct compression comprising the steps of:
a) blending cefditoren pivoxil or a pharmaceutically acceptable salt thereof, lecithin and optionally at least one excipient to form a powder blend without addition of water to form a powder blend;
b) compressing said powder blend into tablets; and then,
c) recovering said tablets.

The excipient may be starch, sucrose, cellulose, dibasic calcium phosphate or a combination thereof. For the practice of the method, at least one additional component such as diluents, lubricants, glidants, disintegrants, preservatives, flavors, antioxidants, sweeteners and combinations thereof may be added in step a).

For any aspect of the invention described above, the lecithin is phosphatidyl choline or a derivative thereof. In addition, the composition may contain other components such as diluents, lubricants, glidants, disintegrants, preservatives, flavors, antioxidants, sweeteners and combinations thereof. The weight ratio of cefditoren pivoxil to lecithin in the composition may be from about 99.9: 0.1 to about 10: 90. A presently preferred weight ratio of cefditoren pivoxil to lecithin is from about 99: 1 to about 1: 2.

### Detailed Description of the Invention

The present invention is directed to a method for enhancing the oral bioavailability of cefditoren pivoxil or a pharmaceutically acceptable salt thereof by formulating the cefditoren pivoxil or a pharmaceutically acceptable salt thereof as a non-emulsified formulation with lecithin; as well as a pharmaceutical composition of cefditoren pivoxil or a pharmaceutically acceptable salt thereof and lecithin in a non-emulsified formulation; the invention is further directed to cefditoren pivoxil or a pharmaceutically acceptable salt thereof for use in a method of treating infections with the non-emulsified formulation, and to a method for preparing tablets by direct compression of blends of cefditoren pivoxil or a pharmaceutically acceptable salt thereof with lecithin. Non-emulsified formulations include solids, tablets, capsules, lozenges, suspensions, elixirs and solutions, and exclude emulsions, liposomes, lipid matrix systems and micro-emulsions. A suitable non-emulsified formulation is a solid formulation.

Detailed discussions of the compositions and methods follow.

### The Compositions

The compositions include cefditoren pivoxil or a pharmaceutically acceptable salt thereof and at least one lecithin in a non-emulsified formulation. Tablets, capsules, lozenges, suspensions, elixirs and solutions are all examples of formulations which are non-emulsified. The tablets may be chewable, effervescent or buccal, for example. The capsules may be hard gelatin capsules or soft elastic gel capsules for example.

Cefditoren is an antibiotic of Formula I, shown below.

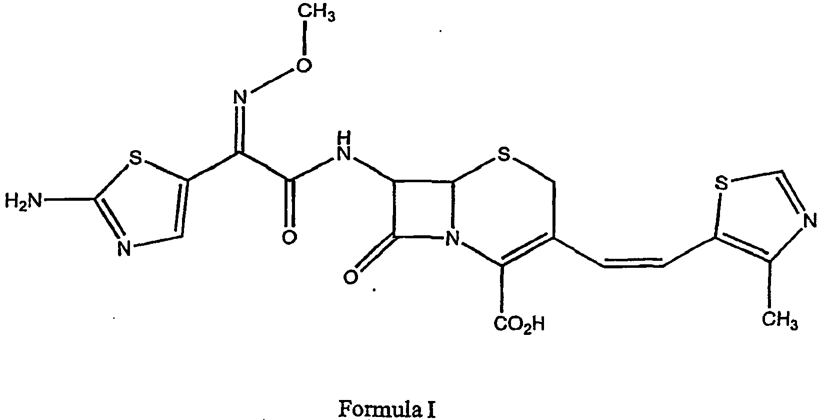

Cefditoren, or (6R,7R)-7-((Z)-2-(2-aminothiazol-4-yl)-2-methoxy-iminoacetamido)-3-((Z)-2-(4-methylthiazol-5-yl)-ethenyl)-8-oxo-5-thia-1-azabicyclo(4.2.0)-oct-2-ene-2-carboxylic acid in chemical nomenclature, is disclosed in U.S. Patent No. 4,839,350. The 2-carboxylic acid can be esterified with a pivaloyloxymethyl group to form cefditoren pivoxil, shown in Formula II below (disclosed in U.S. Patent Nos. 4,839,350 and 4,918,068). The chemical compound is available as a bulk chemical from Meiji Saika Kaisha Ltd of Japan, and a formulation of cefditoren pivoxil with sodium caseinate (disclosed in U.S. Patent No. 5,958,915) is available as in 100 mg tablets under the name of MEIACT from Meiji Saika Kaisha Ltd of Japan.

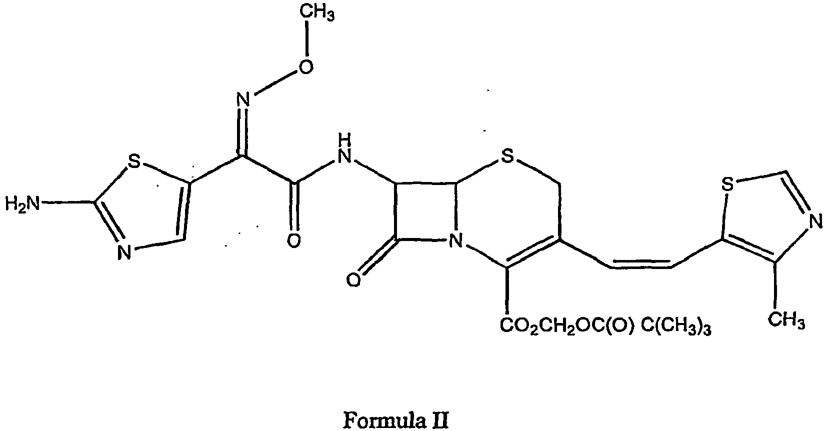

The cephalosporin of Formula II or a pharmaceutically acceptable salt thereof has a broad spectrum of antibacterial activity against gram-negative and gram positive bacteria. This compound exhibits high anti-bacterial activity against *Staphylococcus aureus, Klebsiella pneumoniae* and *Haemophilus influenza* in particular.

The cefditoren pivoxil of the present invention can be used in the form of pharmaceutically acceptable salts derived from inorganic or organic acids. The phrase "pharmaceutically acceptable salt" means those salts which are, within the scope of sound medical judgement, suitable for use in contact with the tissues of humans and lower animals without undue toxicity, irritation, allergic response and the like and are commensurate with a reasonable benefit/risk ratio. Pharmaceutically acceptable salts are well-known in the art. For example, S. M. Berge *et al.* describe pharmaceutically acceptable salts in detail in J. Pharmaceutical Sciences, 1977, 66: 1 et seq*.* The salts can be prepared *in situ* during the final isolation and purification of the compounds of the invention or separately by reacting a free base function with a suitable organic acid. Representative acid addition salts include, but are not limited to acetate, adipate, alginate, citrate, aspartate, benzoate, benzenesulfonate, bisulfate, butyrate, camphorate, camphor sulfonate, digluconate, glycerophosphate, hemisulfate, heptanoate, hexanoate, fumarate, hydrochloride, hydrobromide, hydroiodide, 2-hydroxyethansulfonate (isothionate), lactate, maleate, methane sulfonate, nicotinate, 2-naphthalene sulfonate, oxalate, palmitoate, pectinate, persulfate, 3-phenylpropionate, picrate, pivalate, propionate, succinate, tartrate, thiocyanate, phosphate, glutamate, bicarbonate, p-toluenesulfonate and undecanoate. Also, the basic nitrogen-containing groups can be quaternized with such agents as lower alkyl halides such as methyl, ethyl, propyl, and butyl chlorides, bromides and iodides; dialkyl sulfates like dimethyl, diethyl, dibutyl and diamyl sulfates; long chain halides such as decyl, lauryl, myristyl and stearyl chlorides, bromides and iodides; arylalkyl halides like benzyl and phenethyl bromides and others. Water or oil-soluble or dispersible products are thereby obtained. Examples of acids which can be employed to form pharmaceutically acceptable acid addition salts include such inorganic acids as hydrochloric acid, hydrobromic acid, sulphuric acid and phosphoric acid and such organic acids as oxalic acid, maleic acid, succinic acid and citric acid.

Basic addition salts can be prepared *in situ* during the final isolation and purification of compounds of this invention by reacting a carboxylic acid-containing moiety with a suitable base such as the hydroxide, carbonate or bicarbonate of a pharmaceutically acceptable metal cation or with ammonia or an organic primary, secondary or tertiary amine. Pharmaceutically acceptable salts include, but are not limited to, cations based on alkali metals or alkaline earth metals such as lithium, sodium, potassium, calcium, magnesium and aluminum salts and the like and nontoxic quaternary ammonia and amine cations including ammonium, tetramethylammonium, tetraethylammonium, methylammonium, dimethylammonium, trimethylammonium, triethylammonium, diethylammonium, and ethylammonium among others. Other representative organic amines useful for the formation of base addition salts include ethylenediamine, ethanolamine, diethanolamine, piperidine, piperazine and the like.

The cefditoren pivoxil may be from 10-99.9 weight percent of the formulation and preferably from 80-99 weight percent of the formulation.

The compositions may also contain additional components such as diluents, lubricants, glidants, disintegrants, preservatives, flavors, antioxidants, sweeteners or combinations thereof Suitable diluents include lactose; suitable lubricants include magnesium stearate; suitable glidants include talc; suitable disintegrants include croscarmellose; suitable preservatives include methyl paraben; suitable antioxidants include sodium ascorbate and suitable sweeteners include sucrose and aspartame.

The solid formulation may be granulated and prepared for administration as a suspension in water. When prepared for such administration, the formulation may include additional ingredients such as suspending agents including alginic acid or bentonite among others ; or viscosity enhancers such as guar gum, xanthine gum or carboxymethyl cellulose.

To make the compositions of the present invention, the cefditoren pivoxil or a pharmaceutically acceptable salt thereof is blended or granulated with lecithin; optionally mixed with suitable excipients.
The process does not require any specialized techniques or equipment, in order to formulate the solid, in contrast to the processes required to form liposomes or emulsions. The lecithin in the solid formulations does not serve as an emulsifier or a lipid-forming agent.

An emulsion is a thermodynamic system consisting of at least two immiscible phases; one of which is uniformly dispersed throughout the other as globules. Such system is stabilized with an emulsifying agent.

Micro-emulsions are liquid dispersions of water and oil that are made homogeneous, transparent and stable by the addition of relatively large amounts of surfactants and co-surfactants. To form such dispersions, several components, in specific proportions are required.

Liposomes are sealed sacs dispersed in an aqueous environment. They are micron or sub-micron in size, and have bi-layered walls. The properties and performance of liposomes is highly dependent upon their exact composition and the method of preparation; involving specialized techniques and a number of steps.

In contrast to known emulsions, liposomes or micro-emulsions which include or incorporate lecithin and a drug, the present solid formulations are much simpler, both in terms of constituents and in terms of preparation. Unexpectedly, the solid formulations need contain only cefditoren pivoxil or a pharmaceutically acceptable salt thereof and lecithin in order to achieve their desired effect, although other ingredients may optionally be added. If any other ingredient is added, order of addition or relative proportion is not critical.

Therefore, the phrases "non-emulsified" or "not emulsified" as used herein exclude emulsions, liposomes, lipid matrix systems and micro-emulsions; and include solids, tablets, capsules, lozenges, suspensions, elixirs and solutions.

The amount of lecithin may be in the range of from about 0.1 to about 90 weight percent; and preferably from about 10 to about 60 weight percent.

The lecithin (phosphatidyl choline) may be derived from any source including eggs, soybeans, rapeseed and safflower. Suitable lecithin may be of any grade. A crude, dried lecithin product may be obtained by de-gumming soybean oil. A bleaching agent may be added to clarify and lighten the color of lecithin, which originally is tan-brown color.

Crude lecithin may be extracted with acetone that results in a fine powder or granular product, which process is referred to as de-oiling. Separation of the acetone-soluble fraction increases the amount of phosphatides in the acetone-insoluble fraction by decreasing the amount of triglycerides.

As used herein, the term "lecithin" encompasses phosphatidyl choline obtained naturally or synthetically, including de-oiled or de-gummed products; derivatives of lecithin and combinations of various types of lecithin; since the term lecithin as conventionally used in the art refers to pure phosphatidyl choline and also to crude phospholipid mixtures, containing a variety of other compounds such as fatty acids, triglycerides, sterols, carbohydrates and glycolipids. Commercial lecithin is currently available in more than forty different formulations (from sources such as American Lecithin Co.; Lucas Meyer Inc. and Central Soya Inc. among others) varying from crude oily extracts from natural sources to purified and synthetic phospholipids, all intended to be encompassed by the term "lecithin" as used herein.

Lecithin can be made more hydrophilic by hydroxylation of unsaturated fatty acid constituents, fractionation or compounding with dispersing agents. Moreover, lecithin may be hydroxylated by treating the phosphatides with hydrogen peroxide or peracids in the presence of water-soluble aliphatic carboxylic acids. Alternatively, lecithin may also be hydrolyzed enzymatically to yield a powdered soybean lecithin.

Another lecithin derivative is lysolecithin, which results from the interaction of the enzyme phospholipase with lecithin, for example in pancreatic juices.

Therefore lecithin derivatives are compounds which can be the result of hydroxylation or enzymatic reaction, as mentioned above or other chemical modification of lecithin, included in the broad term "lecithin".

Some examples of suitable lecithins available from Central Soya Inc. of Iowa include BLENDMAX, CENTROBAKE, CENTROCAP, CENTROL CA, CENTROLENE, CENTROMIX, CENTROPHASE, CENTROPHIL, NATHIN and PRECEPT. These names may represent either a single lecithin, or a series of lecithin products, all of which are considered useful for the purposes described herein.

As illustrated by Example 6 below, the order of addition or mixing of ingredients is not critical.

As used herein, the term "composition" is intended to encompass a product comprising the specified ingredients in the specified amounts, as well as any product which results, directly or indirectly, from a combination of the specified ingredients in the specified amounts.

### The Method for Enhancing Oral Bioavailability

A method for enhancing oral bioavailability of cefditoren pivoxil or a pharmaceutically acceptable salt thereof by preparing the cefditoren pivoxil or a pharmaceutically acceptable salt thereof in a solid formulation including lecithin is also disclosed. The term "bioavailability" as used herein refers to a measurement of the rate and extent of therapeutically active drug that reaches the general circulation system. Any systemically-acting orally-administered drug must be absorbed across the gastrointestinal mucosa into the systemic circulation before the drug can demonstrate any therapeutic effect. Certain drugs, including prodrug esters, may undergo degradation in the gastrointestinal tract before they can be systemically absorbed. The greater the degradation, the less drug is available to provide the therapeutic effect. Therefore, any method that enhances the amount of drug reaching the systemic circulation results in higher bioavailability. In the present invention, lecithin retards the rate of degradation (de-esterification) of cefditoren pivoxil. This increases the amount of the drug reaching the systemic circulation which should result in an enhanced therapeutic effect.

As illustrated in Examples 5 and 6 below, cefditoren pivoxil is degraded by esterase enzymes; and lecithin surprisingly and effectively retards such degradation.

The term "prodrug" as used herein represents those prodrugs of the compounds of the present disclosure which are, within the scope of sound medical judgement, suitable for use in contact with the tissues of humans and lower animals without undue toxicity, irritation, allergic response, and the like, commensurate with a reasonable benefit/risk ratio, and effective for their intended use, as well as the zwitterionic forms, where possible, of the compounds of the present disclosure.

Prodrugs of the present disclosure may be rapidly transformed in vivo to the parent compound of the above formula, for example, by hydrolysis in blood. A thorough discussion is provided in T. Higuchi and V. Stella, Pro-drugs as Novel Delivery Systems, V. 14 of the A.C.S. Symposium Series, and in Edward B. Roche, ed., Bioreversible Carriers in Drug Design, American Pharmaceutical Association and Pergamon Press (1987).

Prodrug esters are those prodrugs having ester (-C(O)OR) moieties, where R is an alkyl group or an aryl group.

The term "alkyl" as used herein, alone or in combination, refers to C₁-C₁₂ straight or branched, substituted or unsubstituted saturated chain radicals derived from saturated hydrocarbons by the removal of one hydrogen atom, unless the term alkyl is preceded by a Cₓ-C_{y} designation. Representative examples of alkyl groups include methyl, ethyl, n-propyl, iso-propyl, n-butyl, sec-butyl, iso-butyl, and tert-butyl among others.

The term "aryl" or "aromatic" as used herein alone or in combination refers to a substituted or unsubstituted carbocyclic aromatic group having about 6 to 12 carbon atoms such as phenyl, naphthyl, indenyl, indanyl, azulenyl, fluorenyl and anthracenyl; or a heterocyclic aromatic group which is an aromatic ring containing at least one endocyclic N, O or S atom such as furyl, thienyl, pyridyl, pyrrolyl, oxazolyl, thiazolyl, imidazolyl, pyrazolyl, 2-pyrazolinyl, pyrazolidinyl, isoxazolyl, isothiazolyl, 1,2,3-oxadiazolyl, 1,2,3-triazolyl, 1,3,4-thiadiazolyl, pyridazinyl, pyrimidinyl, pyrazinyl, 1,3,5-triazinyl, 1,3,5-trithianyl, indolizinyl, indolyl, isoindolyl, 3H-indolyl, indolinyl, benzo[b]furanyl, 2,3-dihydrobenzofuranyl, benzo[b]thiophenyl, 1H-indazolyl, benzimidazolyl, benzthiazolyl, purinyl, 4H-quinolizinyl, isoquinolinyl, cinnolinyl, phthalazinyl, quinazolinyl, quinoxalinyl, 1,8-naphthridinyl, pteridinyl, carbazolyl, acridinyl, phenazinyl, phenothiazinyl, phenoxyazinyl, pyrazolo[1,5-c]triazinyl and the like. "Arylalkyl" and "alkylaryl" employ the term "alkyl" as defined above. Rings may be multiply substituted.

A specific example of a prodrug ester is cefditoren pivoxil.

### The Direct Compression Method for Tablet Manufacture

Compressed tablets are the most widely used unit dose oral dosage form. A tablet formulation contains the active drug and certain excipients that either aid the processing or enhance the properties of the active drug product. The drug and the excipients are blended together, wet granulated, processed and compressed into tablets. Alternatively, the tablets may be manufactured by direct compression.

Direct compression of the tablets is the process by which tablets are directly compressed from powder blends of active ingredients and other suitable excipients. This blend should flow uniformly into a die cavity and form into a firm compact upon being compressed. The primary advantages of this process over tablets made by wet granulation include lower production cost and no exposure to heat or moisture, which is particularly important as many drugs are degraded by heat and humidity.

Filler-binders are a vital component of the powder blend that is directly compressed into tablets. Some examples of filler-binders include: spray-dried lactose, sucrose, micro-crystalline cellulose and dibasic calcium phosphate. Lecithins offer the following benefits as filler-binders for directly compressed tablets, especially when the active drug is lipophilic: 1) good binding properties; 2) good surfactant properties; and 3) good flow and compressibility after modifications.

As exemplified by the formulations of Example 2, lecithins demonstrated excellent binding properties. Disintegrating agents were included in these formulations to ensure that the tablets disintegrated and underwent dissolution to release the drug from the tablet.

The percentage by weight ratio of lecithin in the tablet can vary between 0.5-99 percent, and preferably between 5-75 percent of tablet weight. The proportion of other additives to the tablet (lubricating agents and glidants among others) can be varied depending upon the physico-chemical properties of the drug and the desired drug release properties.

Since lecithin is a good surface-active agent, it can enhance the dispersibility and solubility of a hydrophobic drug, as demonstrated in Example 4. This is significant as increased solubility and dispersibility can result in higher drug bioavailability.

It is desirable that excipients used for direct compression should have good flow properties and compressibility. Lecithin can be modified to further enhance these characteristics. One example of a modified lecithin which is a useful excipient is CENTROLEX FP 40 (available from Central Soya Inc. of Iowa), a food-grade, essentially oil-free, medium tan or yellow lecithin powder that is blended with tribasic tricalcium phosphate for improved flowability. Another example of a modified lecithin which is a useful excipient is ALCOLEC SM (available from American Lecithin Inc. of Connecticut), a blend of maltodextrin and refined lecithin, for improved flow properties and compressibility. *Cefditoren pivoxil or a pharmaceutically acceptable salt thereof for use in a Method for Treating Infections*

The term "infection" as used herein refers to invasion and multiplication of pathogenic microorganisms in a bodily part or tissue; which may produce subsequent tissue injury and progress to overt disease through a variety of cellular and toxic mechanisms. The formulations of the present disclosure may be used to treat both gram-positive and gram-negative infections, such as otitis media, sinusitis and pharyngitis, among others. Since use for treating infections also involves treating the resulting symptoms of pain, swelling and inflammation, "treating infections" refers to stopping the invasion as well as treating associated symptoms.

Additionally, the formulations which may be prepared by the methods of the present disclosure may possess immunosuppressive, anti-microbial, anti-fungal, anti-viral, antiinflammatory, and anti-proliferative activity, and possess the ability to reverse chemotherapeutic drug resistance.

Formulations prepared by the methods of the present disclosure would also find utility for use in the treatment of autoimmune diseases, such as rheumatoid arthritis, Hashimoto's thyroiditis, multiple sclerosis, myasthenia gravis, type I diabetes, uveitis, allergic encephalomyelitis and glomerulonephritis among others.

Further uses include the treatment and prophylaxis of inflammatory and hyperproliferative skin diseases and cutaneous manifestations of immunologically-medicated illnesses, such as psoriasis, atopical dermatitis, and *epidermolysis bullosa.* Further instances where a compound of the disclosure would be useful include various eye diseases (autoimmune and otherwise) such as ocular pemphigus, Scleritis, and Graves'opthalmopathy among others.

The phrase "therapeutically effective amount" of the compound of the disclosure as used herein means a sufficient amount of the compound for use to treat disorders, at a reasonable benefit/risk ratio applicable to any medical treatment. It will be understood, however, that the total daily usage of the compounds and compositions of the present disclosure will be decided by the attending physician within the scope of sound medical judgement. The specific therapeutically effective dose level for any particular patient will depend upon a variety of factors including the disorder being treated and the severity of the disorder; activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts. For example, it is well within the skill of the art to start doses of the compound at levels lower than required to achieve the desired therapeutic effect and to gradually increase the dosage until the desired effect is achieved.

These Examples are presented to describe preferred embodiments and utilities of the invention and are not meant to limit the invention unless otherwise stated in the claims appended hereto.

### Example 1

The compatibility of cefditoren pivoxil and modified lecithin granules was evaluated in the following manner. Cefditoren pivoxil (available from Meiji Saika Kaisha Ltd. of Japan, 10 gm) was mixed with modified lecithin (ALCOLEC SM 100, available from American Lecithin Inc. of Connecticut, 5 gm) in a mortar. Sufficient quantity of distilled water was added to transform this mixture into a coherent mass. This mass was dried in an oven for 18 hours at 32°C. This mass was passed through a size 10 sieve to form granules. Cefditoren pivoxil (10 gm) was also granulated with sodium caseinate (available from New Zealand Milk Products North America of California, 4 gm) using the same procedure as described above. Sodium caseinate is used the current formulation of cefditoren pivoxil (MEIACT, available from Meiji Saika Kaisha Ltd of Japan, containing 100 mg cefditoren pivoxil), and these granules served as a control. Both sets of granules were kept in closed containers at room temperature for 30 days. The stability of these granules was evaluated using HPLC (high pressure liquid chromatography). As a result of this study, it was determined that cefditoren pivoxil and modified lecithin granules were as stable as the control.

### Example 2

Nine representative solid formulations of cefditoren pivoxil were prepared in the following manner. 250 mg of cefditoren pivoxil (available from Meiji Saika Kaisha Ltd of Japan) was mixed with cross-linked sodium carboxymethylcellulose (CROSCARMELLOSE, available from FMC Corp. of PA); sodium starch glycolate (EXPLOTAB, available from Penwest Pharmaceutical Co. of IA); modified lecithin (ALCOLEC SM 100, available from American Lecithin Inc. of Connecticut); enzyme-treated lecithin (PRECEPT 8160, available from Central Soya of Iowa) and sodium lauryl sulfate (available from Sigma-Aldrich) in the amounts indicated in Table 1 below.

**Table 1**

| Representative Cefditoren Pivoxil Tablet Formulations | | | | | | |
|---|---|---|---|---|---|---|
| Formulation | Cefditoren Pivoxil (mg) | Carboxy methyl cellulose (mg) | Modified Lecithin (mg) | Sodium Starch Glycolate (mg) | Sodium Lauryl Sulfate (mg) | Enzyme-Treated Lecithin (mg) |
| 1 | 250 | 200 | 500 | 0 | 30 | 0 |
| 2 | 250 | 200 | 400 | 0 | 30 | 0 |
| 3 | 250 | 200 | 400 | 0 | 30 | 0 |
| 4 | 250 | 200 | 400 | 50 | 30 | 0 |
| 5 | 250 | 200 | 400 | 50 | 30 | 0 |
| 6 | 250 | 200 | 400 | 50 | 30 | 0 |
| 7 | 250 | 0 | 400 | 250 | 30 | 0 |
| 8 | 250 | 0 | 0 | 400 | 30 | 400 |
| 9 | 250 | 200 | 0 | 200 | 30 | 400 |

Each blended powder mixes described above were pressed into tablets by direct compression with a Carver pellet press (13 mm die). The press time was 2 seconds and the pressure required to make the tablets was less than 500 pounds for formulations 4-9; 10 seconds and 2000 pounds for formulation 1; 3 seconds and 1000 pounds for formulation 2 and 2 seconds and 500 pounds for formulation 3.

Table formulations 1 and 2 were extremely hard and excluded from further analysis. To determine how effectively the above formulations dissolved, each of the remaining tablets was tested as follows. Each tablet was placed into a dissolution basket and lowered into a beaker containing 900 ml distilled deionized water and 7 ml of 37.5% hydrochloric acid. The baskets containing the tablets were rotated at 100 rpm for 30 minutes. Formulations 7-9 disintegrated completely in 30 minutes.

The dissolution rate of formulations 7-9 was compared to a control, cefditoren pivoxil tablets (SPECTRACEF, available from TAP Pharmaceutical Products, Inc.) SPECTRACEF tablets are bioequivalent to MEIACT tablets, available from Meiji Saika Kaisha Ltd of Japan, containing 100 mg cefditoren pivoxil. The dissolution test was conducted in peaked vessels by the paddle method (US Pharmacopeia, 24 (1999), rotation speed 100 rpm) in 900 ml of 0.1 N. aqueous hydrochloric acid. The dissolution from tablet formulation 9 was comparable to MEIACT at 30 and 60 minute time intervals.

### Example 3

A formulation for cefditoren pivoxil dry powder for suspension (Formulation 10) is shown in Table 2 below. Cefditoren pivoxil (available from Meiji Saika Kaisha Ltd of Japan), modified lecithin (ALCOLEC SM 100, available from American Lecithin Inc. of Connecticut), hydroxypropyl cellulose (available from Aqualon of Wilmington, Delaware), xanthine gum (available from Kelco of San Diego, California), sucrose and strawberry flavor (available from Givaudan Flavor Corp. of New Jersey) were weighed, blended and transferred to a 100 ml high density polyethylene bottle. Next, water (17 ml) was added to the bottle, and then the bottle was shaken vigorously. Sufficient quantity of water (10 ml) was then added to make the final volume of the suspension up to 50 ml at a concentration of 100 mg/5 ml.

**Table 2**

| Representative Cefditoren Pivoxil Formulation 10 | |
|---|---|
| ingredient | amount (gm) |
| cefditoren pivoxil | 1.3 |
| modified lecithin | 10 |
| hydroxypropyl cellulose | 0.1 |
| xanthine gum | 0.1 |
| sucrose | 10 |
| strawberry flavor | 1 |
| water | q.s. 50 ml |

To determine if the drug suspension was as readily dissolved under acidic conditions (similar to that of the stomach) the following experiment was performed. The dissolution rate of formulation 10 was compared to a control, cefditoren pivoxil tablets (SPECTRACEF, available from TAP Pharmaceutical Products, Inc.) SPECTRACEF tablets are bioequivalent to MEIACT tablets, available from Meiji Saika Kaisha Ltd of Japan, containing 100 mg cefditoren pivoxil. A dissolution test for the formulation was conducted in a peaked vessel by the paddle method (rotation speed 75 rpm) in 900 ml of 0.1 N. aqueous hydrochloric acid. Dissolution of formulation 10 was comparable to MEIACT at 30 and 60 minute time intervals.

### Example 4

The solubility of cefditoren pivoxil in various lecithins was determined as follows. Three types of lecithin were tested: lecithin (LECI-PC 35P available from Traco Labs, IL), modified lecithin (containing 70% maltodextrin and 30% phosphatidyl choline; ALCOLEC SM 100 available from American Lecithin Inc. of CT) and hydroxylated lecithin (CENTROLENE A, available from Central Soya, IN). 5%, 10% and 15% suspensions of each type of lecithin were made up in distilled deionized water to form the test dispersions. Cefditoren pivoxil (available from Meiji Saika Kaisha Ltd of Japan) was added to each test dispersion. Each sample was vortexed for one minute, sonicated for two minutes, vortexed again for one minute and then centrifuged at 2000 rpm for ten minutes at room temperature. The resulting supernatant fraction was collected, filtered through a 0.45 µm filter and analyzed by HPLC.

The solubility of cefditoren pivoxil was determined to be 678.5 µg/ml in aqueous lecithin solution, 868.1 µg/ml in modified lecithin and 715.0 µg/ml in hydroxylated lecithin. Since the solubility of cefditoren pivoxil in water alone was 31.8 µg/ml, each of the lecithins tested clearly improved solubility.

### Example 5

To determine whether cefditoren pivoxil is degraded by esterase, and whether such degradation can be impeded by lecithin, the following experiments were performed.

First, a test was performed to determine whether cefditoren pivoxil is degraded by esterase. 25 mg of cefditoren pivoxil (available from Meiji Saika Kaisha Ltd of Japan) was dissolved in 100 ml of an aqueous solution containing 10% dimethylsulfoxide and 0.1 N. hydrochloric acid to make up an 0.25 mg/ml solution. Carboxylesterase (also referred to as esterase herein) from porcine liver (15 mg protein/ml, 250 units/mg protein, available from Sigma-Aldrich) was dissolved in simulated intestinal fluid (SIF having no added pancreatin, available from Sigma-Aldrich) to make up a solution having 0.5 units/ml.

2 ml of the cefditoren pivoxil solution was then mixed with 2 ml of the esterase solution and 2 ml of simulated intestinal fluid. This mixture was allowed to react for 20 minutes at 37 °C and then the reaction was quenched with 1.0 ml of acetonitrile. This procedure was repeated with esterase at two other concentrations. The amount of cefditoren pivoxil remaining in each of the samples was determined by HPLC, and is listed in Table 3 below. The results show that cefditoren pivoxil is degraded, and that the process is concentration-dependent.

**Table 3**

| Concentration-Dependent Esterase Degradation of Cefditoren Pivoxil | |
|---|---|
| esterase concentration (units/ml) | % cefditoren pivoxil remaining |
| 0.05 | 81.9 |
| 0.5 | 4.5 |
| 5 | 0.3 |

Then, a similar procedure was utilized to determine the effect of lecithin on cefditoren pivoxil degradation (demonstrated in Table 3) by esterase. The experimental procedure was as described above, except that the cefditoren pivoxil was dissolved in 3% lecithin (LECI-PC 35 P, available from Traco Labs of IL).

The amount of cefditoren pivoxil remaining in each of the samples was determined by HPLC, and compared to the amount of cefditoren pivoxil of a control solution. The control solution was 2.0 ml cefditoren pivoxil, 2.0 ml of 10% modified lecithin solution and 2.0 ml simulated intestinal fluid incubated and then quenched as above. The amount of cefditoren pivoxil remaining in the lecithin solution was 57.4% compared to 7% for the control (non-lecithin containing) solution. The results indicate that the de-esterification of cefditoren pivoxil by carboxylesterase is greatly impeded by lecithin.

### Example 6

To determine whether the method by which lecithin is added to the formulation affects the impedence of esterase degradation, the following experiment was performed.

The test solutions were:
a) 250 µg/ml cefditoren pivoxil dissolved in 10% DMSO (dimethyl sulfoxide)/0.01 N HCl;
b) 250 µg/ml cefditoren pivoxil dissolved in 10% DMSO/PEG (polyethylene glycol) 400;
c) 250 µg/ml cefditoren pivoxil dissolved in 10% DMSO/0.01 N HCl and modified lecithin was added to make up a solution containing 10 % weight/volume with respect to modified lecithin;
d) 250 µg/ml cefditoren pivoxil dissolved in 10% DMSO/PEG 400; modified lecithin was added to make up a solution containing 10% weight/volume with respect to modified lecithin;
e) 250 µg/ml cefditoren pivoxil dissolved in 10% modified lecithin solution; and
f) cefditoren pivoxil dissolved in 3% sodium caseinate (100 µg/ml).

The cefditoren pivoxil utilized was obtained from Meiji Saika Kaisha Ltd of Japan, the sodium caseinate was obtained from New Zealand Milk Products North America of California and the modified lecithin utilized was ALCOLEC SM 100, available from American Lecithin Inc. of Connecticut.

The test procedure of Example 5 was utilized to analyze the effect of each of the solutions a-f on esterase degradation. The results of Table 4 indicate that modified lecithin retards esterase-induced degradation (solutions c-e), and that similar effects are achieved whether the drug was solubilized in lecithin first (solution d), or the drug was dissolved in other solvents and lecithin was added subsequently (solution c). Sodium caseinate (solution f) was ineffective in preventing esterase-induced degradation. Moreover, comparison of the results for solution a to solution c and solution b to solution d, reveals that the results are not due to the solvent, but rather to the lecithin.

**Table 4**

| Influence of Various Lecithin-Containing Formulations on Esterase Degradation of Cefditoren Pivoxil | |
|---|---|
| solution | % cefditoren pivoxil remaining |
| a | 21.8 |
| b | 17.5 |
| c | 84.1 |
| d | 81.9 |
| e | 87.3 |
| f | 27.0 |

The present invention is illustrated by way of the foregoing description and examples. The foregoing description is intended as a non-limiting illustration, since many variations will become apparent to those skilled in the art in view thereof. It is intended that all such variations within the scope of the appended claims be embraced thereby.

Changes can be made in the composition, operation and arrangement of the method of the present invention described herein without departing from the scope of the invention as defined in the following claims.

## Claims

1. A non-emulsified formulation of Cefditoren pivoxil or of a pharmaceutically acceptable salt thereof and lecithin for enhancing the oral bioavailability of said cefditoren pivoxil.

2. A solid formulation of cefditoren pivoxil or of a pharmaceutically acceptable salt thereof and lecithin for use in treating infections by administration to a patient in need of such treatment of a therapeutically effective amount of said solid formulation.

3. Use of a cefditoren pivoxil or of a pharmaceutically acceptable salt thereof formulated in a non-emulsified formulation or in a solid formulation with lecithin for manufacturing a medicament for enhancing the oral bioavailability of said cefditoren pivoxil.

4. Use of a solid formulation of cefditoren pivoxil or of a pharmaceutically acceptable salt thereof and lecithin for manufacturing a medicament for treating infections by administration to a patient in need of such treatment of a therapeutically effective amount of said solid formulation.

5. The use of one claims 3 or 4 wherein said formulation further comprises at least one additional component selected from the group consisting of diluents, lubricants, glidants, disintegrants, preservatives, flavors, antioxidants, sweeteners and combinations thereof.

6. A pharmaceutical composition comprising: cefditoren pivoxil or a pharmaceutically acceptable salt thereof; and lecithin, wherein said composition is not emulsified or is a solid.

7. The composition of claim 6 wherein the weight ratio of cefditoren pivoxil to lecithin is from about 99.9:0.1 to about 10:90.

8. The composition of any one of claims 6 or 7 further comprising at least one additional component selected from the group consisting of diluents, lubricants, glidants, disintegrants, preservatives, flavors, antioxidants, sweeteners and combinations thereof.

9. A method for preparing tablets by direct compression comprising the steps of:
a) blending a cefditoren pivoxil or a pharmaceutically acceptable salt thereof, lecithin and optionally at least one excipient to form a powder blend without addition of water to form a powder blend; b) compressing said powder blend into tablets; and then, c) recovering said tablets.

10. The method of claim 9 wherein said excipient is selected from the group consisting of starch, sucrose, cellulose, dibasic calcium phosphate and combinations thereof.

11. The method of claim 10 further comprising adding at least one additional component selected from the group consisting of diluents, lubricants, glidants, disintegrants, preservatives, flavors, antioxidants, sweeteners and combinations thereof in step a).

## Patentansprüche

1. Eine nicht-emulgierte Formulierung von Cefditoren-pivoxil oder einem pharmazeutisch verträglichen Salz davon und Lecithin zur Verbesserung der oralen Bioverfügbarkeit des Cefditoren-pivoxils.

2. Eine feste Formulierung von Cefditoren-pivoxil oder einem pharmazeutisch verträglichen Salz davon und Lecithin zur Verwendung in der Behandlung von Infektionen durch Verabreichung einer therapeutisch wirksamen Menge der festen Formulierung an einen Patienten, der eine solche Behandlung benötigt.

3. Verwendung eines Cefditoren-pivoxils oder eines pharmazeutisch verträglichen Salzes davon, formuliert in einer nicht-emulgierten Formulierung oder in einer festen Formulierung mit Lecithin, zur Herstellung eines Medikaments zur Verbesserung der oralen Bioverfügbarkeit des Cefditoren-pivoxils.

4. Verwendung einer festen Formulierung von Cefditoren-pivoxil oder einem pharmazeutisch verträglichen Salz davon und Lecithin zur Herstellung eines Medikaments für die Behandlung von Infektionen durch Verabreichung einer therapeutisch wirksamen Menge der festen Formulierung an einen Patienten, der eine solche Behandlung benötigt.

5. Die Verwendung gemäß einem der Ansprüche 3 oder 4, worin die Formulierung weiter mindestens einen zusätzlichen Bestandteil umfasst, gewählt aus der Gruppe bestehend aus Verdünnungsmitteln, Schmiermitteln, Flussregulierungsmitteln, Desintegrationsmitteln, Konservierungsstoffen, Geschmacksstoffen, Antioxidantien, Süßstoffen und Kombinationen davon.

6. Eine pharmazeutische Zusammensetzung, die Folgendes umfasst: Cefditoren-pivoxil oder ein pharmazeutisch verträgliches Salz davon; und Lecithin, worin die Zusammensetzung nicht-emulgiert ist oder ein Feststoff ist.

7. Die Zusammensetzung gemäß Anspruch 6, worin das Gewichtsverhältnis von Cefditoren-pivoxil zu Lecithin von ungefähr 99,9:0,1 bis ungefähr 10:90 beträgt.

8. Die Zusammensetzung gemäß einem beliebigen der Ansprüche 6 oder 7, die weiter mindestens einen zusätzlichen Bestandteil umfasst, gewählt aus der Gruppe bestehend aus Verdünnungsmitteln, Schmiermitteln, Flussregulierungsmitteln, Desintegrationsmitteln, Konservierungsstoffen, Geschmacksstoffen, Antioxidantien, Süßstoffen und Kombinationen davon.

9. Ein Verfahren zur Herstellung von Tabletten durch direkte Kompression, das folgende Schritte umfasst:
a) Mischen von einem Cefditoren-pivoxil oder einem pharmazeutisch verträglichen Salz davon, Lecithin und wahlweise mindestens einem Arzneistoffträger, um eine Pulvermischung zu bilden ohne Hinzugabe von Wasser, um eine Pulvermischung zu bilden; b) Komprimieren der Pulvermischung zu Tabletten; und dann c) Gewinnen der Tabletten.

10. Das Verfahren gemäß Anspruch 9, worin der Arzneistoffträger gewählt ist aus der Gruppe bestehend aus Stärke, Saccharose, Cellulose, zweibasigem Kalziumphosphat und Kombinationen davon.

11. Das Verfahren gemäß Anspruch 10, das weiter das Hinzufügen mindestens eines zusätzlichen Bestandteils, der gewählt ist aus der Gruppe bestehend aus Verdünnungsmitteln, Schmiermitteln, Flussregulierungsmitteln, Desintegrationsmitteln, Konservierungsstoffen, Geschmacksstoffen, Antioxidantien, Süßstoffen und Kombinationen davon, in Schritt a) umfasst.

## Revendications

1. Formulation non émulsionnée de Cefditorène pivoxil ou d'un de ses sels pharmaceutiquement acceptables et de lécithine pour améliorer la biodisponibilité orale dudit Cefditorène pivoxil.

2. Formulation solide de Cefditorène pivoxil ou d'un de ses sels pharmaceutiquement acceptables et de lécithine pour une utilisation dans le traitement des infections par l'administration à un patient qui a besoin d'un tel traitement d'une quantité thérapeutiquement efficace de ladite formulation solide.

3. Utilisation d'un Cefditorène pivoxil ou d'un de ses sels pharmaceutiquement acceptables formulé en une formulation non émulsionnée ou en une formulation solide avec de la lécithine pour la fabrication d'un médicament destiné à améliorer la biodisponibilité orale dudit Cefditorène pivoxil.

4. Utilisation d'une formulation solide de Cefditorène pivoxil ou d'un de ses sels pharmaceutiquement acceptables et de la lécithine pour la fabrication d'un médicament destiné au traitement des infections par l'administration à un patient qui a besoin d'un tel traitement d'une quantité thérapeutiquement efficace de ladite formulation solide.

5. Utilisation d'une des revendications 3 ou 4, dans laquelle ladite formulation comprend en outre au moins un composant additionnel choisi dans le groupe constitué par des diluants, des lubrifiants, des agents de glissement, des agents de désintégration, des conservateurs, des arômes, des antioxydants, des édulcorants et des combinaisons de ceux-ci.

6. Composition pharmaceutique comprenant : du Cefditorène pivoxil ou un de ses sels pharmaceutiquement acceptables ; et de la lécithine, dans laquelle ladite composition n'est pas émulsionnée ou est un solide.

7. Composition selon la revendication 6, dans laquelle le rapport en poids du Cefditorène pivoxil sur la lécithine est d'environ 99,9 : 0,1 à environ 10 : 90.

8. Composition selon l'une quelconque des revendications 6 ou 7, comprenant en outre au moins un composant additionnel choisi dans le groupe constitué des diluants, des lubrifiants, des agents de glissement, des agents de désintégration, des conservateurs, des arômes, des antioxydants, des édulcorants et des combinaisons de ceux-ci.

9. Procédé de préparation de comprimés par compression directe, comprenant les étapes consistant à : a) mélanger un Cefditorène pivoxil ou un de ses sels pharmaceutiquement acceptables, de la lécithine et éventuellement au moins un excipient pour former un mélange pulvérulent sans addition d'eau dans le but de former un mélange pulvérulent ; b) comprimer ledit mélange pulvérulent en comprimés ; et puis, c) récupérer lesdits comprimés.

10. Procédé selon la revendication 9, dans lequel ledit excipient est choisi dans le groupe constitué par l'amidon, le saccharose, la cellulose, le phosphate de calcium dibasique et des combinaisons de ceux-ci.

11. Procédé selon la revendication 10, comprenant en outre l'addition d'au moins un composant additionnel choisi dans le groupe constitué des diluants, des lubrifiants, des agents de glissement, des agents de désintégration, des conservateurs, des arômes, des antioxydants, des édulcorants et des combinaisons de ceux-ci à l'étape a).
